(19) Europäisches Patentamt
European Patent Office
Office européen des brevets

(11) Veröffentlichungsnummer: **0 508 038 B1**

# (12) EUROPÄISCHE PATENTSCHRIFT

(45) Veröffentlichungstag der Patentschrift: **22.03.95**

(51) Int. Cl.[6]: **C07C 309/17**, A61K 7/075, A61K 7/50, C11D 1/12

(21) Anmeldenummer: **92100255.6**

(22) Anmeldetag: **08.01.92**

## (54) Citronensäurefettalkoholesterpolyglykolethersulfosuccinate, Verfahren zu ihrer Herstellung und ihre Verwendung.

(30) Priorität: **11.04.91 DE 4111811**

(43) Veröffentlichungstag der Anmeldung:
**14.10.92 Patentblatt 92/42**

(45) Bekanntmachung des Hinweises auf die Patenterteilung:
**22.03.95 Patentblatt 95/12**

(84) Benannte Vertragsstaaten:
**AT BE CH DE DK ES FR GB GR IT LI LU NL SE**

(56) Entgegenhaltungen:
**EP-A- 0 334 482**
**DE-A- 2 700 072**
**US-A- 4 866 203**

(73) Patentinhaber: **Witco Surfactants GmbH**
**Industriegebiet West**
**D-36396 Steinau an der Strasse (DE)**

(72) Erfinder: **Hohn, Elke, Dipl.-Chem.**
**Struthrain 20**
**W-6490 Schlüchtern (DE)**
Erfinder: **Köhle, Hans-Jürgen, Dr. Dipl.-Chem.**
**Lotichiusstrasse 13**
**W-6490 Schlüchtern (DE)**
Erfinder: **Urban, Georg-Friedrich, Ing. Grad.**
**Armin-Strasse 90**
**W-6140 Bensheim (DE)**
Erfinder: **Weigand, Joachim, Dr. Dipl.-Chem.**
**Hauptstrasse 71**
**W-6463 Freigericht 1 (DE)**
Erfinder: **Möller, Christl**
**Bergwinkelstrasse 2**
**W-6497 Steinau an der Strasse (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Gegenstand der Erfindung sind neue Citronensäurefettalkoholesterpolyglykolethersulfosuccinate, Verfahren zu ihrer Herstellung und ihre Verwendung als Reinigungsmittel und für kosmetische Präparate.

Kosmetische Präparate, insbesondere solche, die in den Bereich der Haar- und Körperreinigung fallen, wie Duschbäder, Schaumbäder, Haarshampoos, flüssige Seifen, enthalten als Reinigungskomponenten hauptsächlich Aniontenside wie Carboxylate, Alkylsulfate und Alkylethersulfate, Sulfosuccinate.

Diese Zubereitungen sollen die Hautoberfläche reinigen, vorzugsweise nur den auf ihr anhaftenden Film, welcher aus Körperausscheidungen wie Schweiß und Fetten, Hautschuppen oder abgelagertem Schmutz aus der Umgebung bestehen kann. Die Reinigungsmittel sollen keinesfalls die Haut auslaugen, sie reizen oder ihre natürliche Funktion beeinträchtigen.

Da die Präparate aber bei ihrer häufigen - in den letzten Jahren fast täglichen - Anwendung zu Irritationen der Haut führen können, werden zur Verbesserung der Haut- und Augenschleimhautverträglichkeit häufig sogenannte milde Tenside mitverwendet, wie beispielsweise Betaine, Proteinderivate, Ampholyte, Alkylethercarboxylate und Sulfosuccinate.

Insbesondere für Babypflegemittel und Babyshampoos wird besonderer Wert auf extrem niedrige Gehalte an die Haut und Augenschleimhaut reizende Substanzen gelegt. Die konventionell wegen ihrer ausgezeichneten Reinigigungs- und Schaumbildungseigenschaften verwendeten anionischen Tenside können durch die bekannten milden Co-Tenside in ihrer Irritationswirkung zwar weitgehend gemildert werden, in der Praxis werden aber noch Verbesserungen insbesondere in Richtung auf Augenschleimhautverträglichkeit gefordert.

In hohen Konzentrationen oder allein sind die milden Tenside zwar weitgehend irritationsfrei, zeigen dann jedoch keine praxisgerechten Schäumungs- und Reinigungseigenschaften und unbefriedigende Viskositäten.

Ein weiteres Kriterium für die Brauchbarkeit der oberflächenaktiven Substanzen ist ihre Toxizität. Die Toxizität liegt in den Tensiden selbst oder ihren durch Wechselwirkung mit den Bestandteilen der Rezeptur entstehenden Produkten.

So werden die wegen ihrer vorteilhaften anwendungstechnischen und hautpflegenden Eigenschaften bislang gern mitverwendeten Fettsäurealkanolamide in letzter Zeit nur noch ungern in kosmetischen Präparaten eingesetzt, da der bei der Herstellung verbleibende Restgehalt an freiem Diethanolamin nicht ausgeschlossen werden und damit Anlaß zur Nitrosaminbildung sein kann (EP-A-0 306 843). N-Nitrosamine wirken im Tierversuch krebserzeugend.

Aufgrund des gewachsenen Umweltbewußtseins werden weiterhin Produkte verlangt, welche auf natürlichen nachwachsenden Rohstoffen basieren, keinerlei Toxizität aufweisen und in den kommunalen Kläranlagen schnell und vollständig ohne toxische Zwischenprodukte abgebaut werden können.

Die in der Praxis eingeführten Produkte auf Basis von Citronensäure wie beispielsweise die Mono- und/oder Difettsäureesteralkoxylate auf Basis von Glycerin weisen zwar niedrige Toxizitäten auf und sind milde die Haut nicht reizende Produkte, welche hinsichtlich ihrer Reinigungswirkung oder Löslichkeit aber nicht den Erfordernissen der Praxis entsprechen.

Aufgabe der vorliegenden Erfindung ist es, diese Nachteile des Standes der Technik zu überwinden und milde, hautfreundliche Reinigungsmittel für Haushalt und Industrie und kosmetische Präparate, insbesondere solche, die in den Bereich der Haar- und Körperreinigung fallen, zur Verfügung zu stellen.

Diese Aufgabe wird gelöst durch die erfindungsgemäß mitverwendeten alkoxylierte Estergruppen enthaltenden Sulfosuccinate auf Basis von Citronensäure. Gegenstand der Erfindung sind Citronensäurefettalkoholesterpolyglykolethersulfosuccinate der allgemeinen Formel (1)

$$\begin{array}{l} \quad H_2C-COO-(CHR-CH_2-O)_a-R^1 \\ \qquad | \\ HO-C-COO(CHR-CH_2-O)_b-R^2 \\ \qquad | \\ \quad H_2C-COO(CHR-CH_2-O)_c-R^3 \end{array} \qquad (1)$$

worin

R = gleich oder verschieden H oder $-CH_3$

$R^1$ =

$$-CO-\underset{\displaystyle SO_3^-\,x^+}{\underset{|}{CH}}-CH_2-COO^-\,x^+ \quad \text{oder} \quad -CO-CH_2-\underset{\displaystyle SO_3^-\,x^+}{\underset{|}{CH}}-COO^-\,x^+$$

$R^2$ = $R^1$ oder H oder $R^3$

$R^3$ = ein gegebenenfalls substituierter und/oder Mehrfachbindungen enthaltender Alkyl- oder Acylrest mit 8 - 22 C-Atomen, vorzugsweise 12 - 18 C-Atomen

a,b = gleich oder verschieden 0 - 5, vorzugsweise 1 - 3, sein kann, wobei a + b ≧1, vorzugsweise ≧2, insbesondere im Bereich von 2 - 5, liegt und

c = 0 - 15, vorzugsweise 1 - 10, insbesondere

$x^+$ = $H^+$ oder ein Kation ist.

Ein weiterer Gegenstand der Erfindung ist ein Verfahren zur Herstellung von Verbindungen der allgemeinen Formel (1).

Weitere Gegenstände der Erfindung sind wässrige Haarreinigungs- und Pflegemittel, Spülmittel, Allzweckreiniger, Neutralreiniger, Hautpflege- und reinigungsmittel, welche dadurch gekennzeichnet sind, daß sie 1 - 10, vorzugsweise 2 - 8 Gew.-Teile mindestens eine der Verbindungen der allgemeinen Formel (1), definierte Mengen mindestens eines Tensids aus der Gruppe der nichtionischen, amphoteren, zwitterionischen und ionischen Tenside, vorzugsweise 0,1 bis 30 Gew.-Teile, gegebenenfalls 0,1 bis 15 Gew.-Teile einer der üblichen Mittel aus der Gruppe der Zusatz- und Hilfsstoffe, Verdickungsmittel, Duftstoffe, Konservierungsmittel, Farbstoffe, Pflanzenextrakte und gegebenenfalls ad 100 Wasser enthalten.

Weitere Gegenstände der Erfindung sind durch die Ansprüche gekennzeichnet.

Ein Ausgangsstoff für die Herstellung der erfindungsgemäßen Verbindungen der allgemeinen Formel (1) ist natürliche oder synthetische Citronensäure. Je nach Anforderungen an das Endprodukt kann hier hochgereinigte Qualität oder die handelsübliche Ware von technischer Reinheit eingesetzt werden.

Die Citronensäure kann in einem ersten Schritt mit einem bzw. zwei Mol Fettalkohol bzw. Fettalkoholalkoxylat nach den bekannten Methoden zum Ester umgesetzt werden (US-PS 2 076 111, US-PS 3 948 976, EP 0 199 131). Erfindungsgemäß bevorzugt sind die Monoester.

Als Alkohole werden die monofunktionellen Fettalkohole mit 8 - 22 C-Atomen - bevorzugt die natürlich vorkommenden - verwendet, sowie die Fettalkohole, welche nach den bekannten Verfahren aus den synthetischen oder natürlichen Fettsäuren hergestellt werden können, wie Laurylalkohol, Palmitylalkohol, Stearylalkohol, Oleylalkohol, Linolylalkohol, Talgalkohol und die Alkohole auf Basis der natürlichen Kokosfettsäuren.

Synthetische Alkohole, wie z. B. die nach der Oxosynthese oder nach der Guerbet-Reaktion hergestellten Verbindungen, sind ebenfalls verwendbar.

Die Alkohole können als solche oder in Form der daraus nach bekannten Verfahren alkoxylierten, vorzugsweise ethoxylierten und/oder propoxylierten, mindestens eine Ethergruppe enthaltenden Alkohole eingesetzt werden.

Erfindungsgemäß bevorzugt werden Alkoxylierungsprodukte mit C = 1 - 15, vorzugsweise 1 - 10 und 11 Alkoxyeinheiten/Mol Fettalkohol (US-PS 4 866 203 und die dort zitierten Literaturstellen).

Anstelle der Fettalkoholalkoxylate können auch die nach bekannten Verfahren alkoxylierten, vorzugsweise ethoxylierten und/oder propoxylierten Fettsäuren eingesetzt werden (EP-A-0 334 482).

Die Alkoxylierungsgrade stimmen mit denen der Fettalkoholalkoxylate überein. In diesen Produkten ist $R^3$ der allgemeinen Formel 1 ein Acylrest.

Als Fettsäuren werden die einbasischen Säuren mit 8 - 22 C-Atomen - bevorzugt die natürlich vorkommenden - verwendet wie Laurinsäure, Palmitinsäure, Stearinsäure, Olsäure, Linolsäure, Linolensäure, Ricinolsäure, Kokosfettsäure oder deren Mischungen.

Diese Ester werden in einem zweiten Schritt nach an sich bekannten Verfahren (J. Am. Oil Chemist's Soc., 1956, 33, 571, EP-A-0 334 482) mit Alkylenoxiden wie insbesondere Ethylenoxid und Propylenoxid zu den Polyglykoletherestern umgesetzt, wobei im statistischen Mittel pro Carboxylgruppe 0 - 5, vorzugsweise 1 - 3, Mol Alkylenoxid angelagert werden. Es wurde gefunden, daß die erfindungsgemäßen Produkte mit insgesamt (a + b der allgemeinen Formel 1) ≧1, vorzugsweise ≧2 Mol Alkylenoxid ausgezeichnete Ergebnisse liefern. In erfindungsgemäß besonders bevorzugten Produkten liegt a + b zwischen 2 und 5.

Die Citronensäure kann in erster Stufe auch noch nach den oben angeführten bekannten Verfahren alkoxyliert und in zweiter Stufe mit 1 oder 2 Mol Fettsäure zum Fettsäureester umgesetzt werden, wobei $R^3$ ebenfalls ein Acylrest ist und a + b + c = 1 - 25, vorzugsweise 5 - 15 und insbesondere 3 - 5, sind.

Die Reaktion zum Sulfosuccinat erfolgt mit Maleinsäureanhydrid und anschließender Sulfierung mit Natriumsulfit nach an sich bekannten Verfahren (DE-OS 2 700 072).

Dabei wird pro Mol umzusetzende Hydroxylgruppe mit einem Mol Maleinsäureanhydrid bei 60 - 80 °C bis zur vollständigen Reaktion des Anhydrids gerührt. Der Maleinsäurehalbester wird anschließend in eine wässrige Natriumsulfit-Lösung gegeben (1 Equivalent Sulfit pro Equivalent Halbester) und bei 60 - 80 °C sulfiert, bis das Sulfit vollständig abreagiert ist. Dann wird das wässrige Produkt pH-neutral eingestellt.

Im allgemeinen wird erfindungsgemäß das Verfahren so geführt, daß in erster Stufe die Citronensäuremonoester mit dem Fettalkohol bzw. dem Fettalkoholalkoxylat, gegebenenfalls unter Mitverwendung eines Veresterungskatalysators, bei 140 - 160 °C unter Entfernung des Reaktionswassers verestert, dieser in zweiter Stufe in Gegenwart eines basischen Katalysators bei 80 - 150 °C und 1 - 10 bar mit Alkylenoxid zum Alkoxylat umgesetzt wird, welches in dritter Stufe durch Addition von Maleinsäureanhydrid bei 60 - 80 °C zum Halbester und in letzter Stufe bei 60 - 80 °C mit wässriger Natriumsulfitlösung zum Salz der entsprechenden Sulfonsäure umgesetzt wird.

Die erfindungsgemäßen Verbindungen sind durch die allgemeine Formel (1) in idealisierter Form dargestellt. Die technischen Mischungen enthalten neben den angegebenen Monofettsäureestern oder Difettsäureestern noch geringe Anteile der höheren Ester, und auch die angegebenen Alkoxylierungsgrade sind statistische Mittelwerte.

Für die erfindungsgemäße Verwendung ist eine Reinigung nicht erforderlich, das heißt, die technischen Mischungen sind als solche direkt weiter verwendbar.

Die erfindungsgemäßen Mischungen für Reinigungszwecke und Kosmetik liegen im allgemeinen als wässrige Mittel oder als wässrige alkoholische Lösungen, Cremes, Emulsion, Gele vor und können zur Anpassung an den vorgesehenen Anwendungszweck noch die jeweils üblichen Hilfs- und Zusatzstoffe enthalten, welche für die Herstellung von Reinigungsmitteln und kosmetischen Präparaten im Bereich der Haar- und Körperreinigung, das heißt für Duschbäder, Schaumbäder, Shampoos, flüssige Seifen, Babypflege- und waschmittel sowie für milde Reinigungsmittel wie Geschirrspülmittel, Allzweckreiniger oder Neutralreiniger für manuelle Anwendung üblich sind, mitverwendet werden.

Für die kosmetischen Anwendungen können die auf diesen Gebieten üblichen Tenside, Duftstoffe, Konservierungsmittel, Farbstoffe, Pflanzenextrakte bzw. sonstige kosmetische Additive mitverwendet werden.

Als Tenside kommen neben den bekannten Betainen, amphoteren und nichtionischen Verbindungen für die reinigenden Rezepturen insbesondere die anionischen Tenside wie Carboxylate, Alkylsulfate, Alkylethersulfate, Alkylsulfonate, Alkylethersulfonate, Alkylsulfosuccinate in Betracht. Erfindungsgemäß bevorzugt werden Alkylamidobetaine wie REWOTERIC[R 1)] AM B 13 und AM B 14, Carboxyglycinate wie REWOTERIC[R] AM 2 C NM, Carboxypropionate wie REWOTERIC[R] AM KSF 40, Sulfobetaine wie REWOTERIC[R] AM CAS, anionische Tenside wie Ethersulfate REWOPOL[R 2)] NL 3, Ethercarboxylate wie REWOPOL[R] CLN 100, Sulfosuccinate wie REWOPOL[R] SB FA 30, REWOPOL[R] SBZ, REWODERM[R 3)] SPS, nichtionische Tenside wie Glycerinfettsäureesterethoxylate wie REWODERM[R] ES 90, Glycerinmonostearat wie REWOMUL[R 4)] MG, Cetylalkohol ([1)], [2)], [3)], [4)] = Warenzeichen der Fa. REWO Chemische Werke GmbH, Steinau an der Straße).

Neben den erfindungsgemäßen Verbindungen, welche in Mengen von 1 - 10 Gew.-Teilen, insbesondere 2 - 8 Gew.-Teilen, bei Shampoos und 1 - 7 Gew.-Teilen, vorzugsweise 1 - 5 Gew.-Teilen, bei Cremes sowie 1 - 5, vorzugsweise 1,5 - 3 Gew.-Teilen bei Reinigungsmitteln eingesetzt werden, werden die anderen Tenside bei den Shampoos üblicherweise in Mengen von 1 - 20 Gew.-Teilen, vorzugsweise 5 - 15 Gew.-Teilen, bei den Cremes in Mengen von 1 - 10 Gew.-Teilen, vorzugsweise 2 - 5 Gew.-Teilen, mitverwendet.

Als Verdickungsmittel werden 1 - 8 Gew.-Teile der auf diesem Gebiet üblichen Verbindungen wie Glycerinfettsäureesterethoxylate, Fettalkoholethoxylate, Fettsäurealkylolamide und die üblichen Alkali-, Erdalkali- und Ammoniumsalze, welche bei 20 °C in Mengen von mindestens 1 Gew.-% in Wasser löslich sind, wie insbesondere NaCl, $NH_4Cl$ eingesetzt.

Die in den nachfolgenden Beispielen angeführten Analysenwerte werden nach den folgenden auf diesem Gebiet allgemein üblichen Methoden bestimmt:

Säurezahl (SZ)

Die Säurezahl ist ein Maß für den Gehalt eines Fettes oder von technischen Fettsäuren an freien Säuren und gibt die Milligramm Kaliumhydroxid an, die notwendig sind, um 1 Gramm Substanz zu neutralisieren.

Die Werte werden bestimmt nach der DGF-Einheitsmethode C-V4.

Gehalt an Trockensubstanz

Der Gehalt wird bestimmt durch Erhitzen und Trocknen bei 105 °C bis zur Gewichtskonstanz.

Hydroxylzahl (OHZ)

Die Hydroxylzahl dient zur Ermittlung des Gehalts an Hydroxylgruppen und gibt die Anzahl Milligramm Kaliumhydroxid an, die notwendig ist, um die von 1 Gramm Fett bei der Acetylierung verbrauchte Essigsäure zu neutralisieren (mg KOH/g).

Benzavlon-WAS

Der in den nachfolgenden Beispielen angegebene Gehalt an waschaktiver Substanz (B-WAS) wird durch die übliche Zweiphasentitration mit Benzalkoniumchlorid gegen Methylenblau als Indikator titriert (vgl. S. R. Apton, Nature (London) 160, 1967, S. 795).

Herstellung der erfindungsgemäßen Verbindungen

Beispiel 1

1.1 Veresterung

377 g (1,79 mol) Citronensäure Monohydrat, 723,6 g (1,97 mol) REWOPAL[R] LA 4 *und 1,3 g Hypophosphorige Säure wurden unter Stickstoff in ein Reaktionsgefäß, ausgerüstet mit Rührer, Thermometer, einem Wasserabscheider und Kühler, eingefüllt.

Die Mischung wurde unter $N_2$-Atmosphäre und gutem Rühren auf 140 °C aufgeheizt und 2 h bei dieser Temperatur gehalten. Während dieser Zeit destillierte nahezu die theoretische Menge des Reaktionswassers ab. Nachdem die Reaktionsmischung auf 80 °C abgekühlt war, wurde der Druck langsam auf 20 mbar reduziert und die Reaktion bei diesem Druck und 140 °C vervollständigt.

Das Reaktionsprodukt hat eine Säurezahl von 200.

1.2 Ethoxilierung

784 g Citronensäuremonoester aus Beispiel 1.1 wurden nach Zusatz von 4 g Alkyldimethylamin bei 90 °C im Autoklaven portionsweise mit 119 g Ethylenoxid umgesetzt, so daß der Druck maximal 5 bar betrug.

| Analysendaten: | |
|---|---|
| Säurezahl<br>Hydroxylzahl | 24,6 mg KOH/g<br>187,3 mg KOH/g |

1.3 Halbester-Bildung

333 g (1,2 mol) Ethoxylat aus Beispiel 1.2 wurden bei 70 - 80 °C unter $N_2$ mit 98 g (1,0 mol) Maleinsäureanhydrid versetzt und 2 h bei dieser Temperatur gerührt. Das Reaktionsprodukt hatte die folgenden Analysenzahlen:

| | |
|---|---|
| Säurezahl in Aceton<br>Säurezahl in Isopropanol | 148,2 mg KOH/g<br>140,2 mg KOH/g |

* = Produkt und Warenzeichen der Fa. REWO Chemische Werke GmbH; $C_{12/14}$ Fettalkoholethoxylat mit 4 Ethylenoxideinheiten (EO) und einer OHZ von 153.

1.4 Sulfierung

Zu einer Lösung von 108 g (1,0 mol) Natriumsulfit in 680 g Wasser wurden bei 60 - 70 °C 350 g Halbester aus Beispiel 1.3 bei dieser Temperatur langsam zugetropft. Die Lösung wies folgende Analysendaten auf:

| | |
|---|---|
| Trockenrückstand | 40,4 % |
| Benzavlon-WAS | 34,3 % |
| pH-Wert (5 %) | 6.1 |

Beispiel 2

2.1 Veresterung

210 g (1 mol) Citronensäure Monohydrat, 507 g (1,1 mol) REWOPAL$^R$ LA 6*und 1,3 g hypophosphorige Säure wurden unter $N_2$ in ein Reaktionsgefäß, ausgerüstet mit Rührer, Thermometer, einem Wasserabscheider und Kühler eingefüllt.

Die Mischung wurde unter einer Stickstoffatmosphäre und gutem Rühren auf 140 °C erhitzt und 2 h bei dieser Temperatur gehalten. Während dieser Zeit destillierte nahezu die theoretische Menge des Reaktionswassers ab. Man ließ die Reaktionsmischung auf 80 °C abkühlen und legte ein Vakuum von 20 mbar an, um die Umsetzung zu vervollständigen. Die Säurezahl des Endprodukts lag bei 173.

2.2 Ethoxylierung

610 g Citronensäuremonoester aus Beispiel 2.1 wurden nach Zusatz von 3 g Alkyldimethylamin bei 90 °C im Autoklaven portionsweise mit 116 g Ethylenoxid umgesetzt, so daß der Druck maximal 5 bar betrug.

| Analysendaten: | |
|---|---|
| Säurezahl | 6,7 mg KOH/g |
| Hydroxylzahl | 185 mg KOH/g |

2.3 Halbester-Bildung

363 g (1,2 mol) Ethoxylat aus Beispiel 2.2 wurden bei 70 - 80 °C unter $N_2$ mit 98 g (1 mol) Maleinsäureanhydrid versetzt und 2 h bei dieser Temperatur gerührt. Das Reaktionsprodukt hatte die folgenden Analysenzahlen:

| | |
|---|---|
| Säurezahl in Aceton | 150 mg KOH/g |
| Säurezahl in Isopropanol | 137 mg KOH/g |

2.4 Sulfierung

Zu einer Lösung von 101 g (1 mol) Natriumsulfit in 680 g Wasser wurden bei 60 - 70 °C 350 g Halbester aus Beispiel 2.3 bei dieser Temperatur langsam zugetropft. Die Lösung wies folgende Analysendaten auf:

* = Produkt und Warenzeichen der Fa. REWO Chemische Werke GmbH; $C_{12/14}$ Fettalkoholethoxylat mit 6 Ethylenoxideinheiten (EO) und einer OHZ von 130.

| | |
|---|---|
| Trockenrückstand | 39,7 % |
| Benzavlon-WAS | 28,4 % |
| pH-Wert (5 %) | 6,3 |

Beispiel 3

3.1 Veresterung

141 g (0,67 mol) Citronensäure Monohydrat, 470 g (0,73 mol) REWOPAL® LA 10*und 1 g hypophosphorige Säure wurden unter $N_2$ in ein Reaktionsgefäß, ausgerüstet mit Rührer, Thermometer, einem Wasserabscheider und Kühler eingefüllt.

Die Mischung wurde unter einer Stickstoffatmosphäre und gutem Rühren auf 140 °C erhitzt und 2 h bei dieser Temperatur gehalten. Während dieser Zeit destillierte nahezu die theoretische Menge des Reaktionswassers ab (Säurezahl des Produkts: 121). Man ließ die Reaktionsmischung auf 80 °C abkühlen und legte ein Vakuum von 20 mbar an, um die Umsetzung zu vervollständigen. Die Säurezahl des Endprodukts lag bei 113.

3.2 Ethoxylierung

513 g Citronensäuremonoester aus Beispiel 3.1 wurden nach Zusatz von 2,6 g Alkyldimethylamin bei 90 °C im Autoklaven portionsweise mit 60 g Ethylenoxid umgesetzt, so daß der Druck maximal 5 bar betrug.

| Analysendaten: | |
|---|---|
| Säurezahl | 2,7 mg KOH/g |
| Hydroxylzahl | 108 mg KOH/g |

3.3 Halbester-Bildung

478 g (0,9 mol) Ethoxylat aus Beispiel 3.2 wurden bei 70 - 80 °C unter $N_2$ mit 75 g (0,8 mol) Maleinsäureanhydrid versetzt und 2 h bei dieser Temperatur gerührt. Das Reaktionsprodukt hatte die folgenden Analysenzahlen:

| | |
|---|---|
| Säurezahl in Aceton | 89,3 mg KOH/g |
| Säurezahl in Isopropanol | 82,8 mg KOH/g |

3.4 Sulfierung

Zu einer Lösung von 89 g (0,8 mol) Natriumsulfit in 850 g Wasser wurden bei 60 - 70 °C 480 g Halbester aus Beispiel 3.3 bei dieser Temperatur langsam zugetropft. Die Lösung wies folgende Analysendaten auf:

| | |
|---|---|
| Trockenrückstand | 39,2 % |
| Benzavlon-WAS | 28,9 % |
| pH-Wert (5 %) | 6,1 |

* = Produkt und Warenzeichen der Fa. REWO Chemische Werke GmbH; $C_{12/14}$ Fettalkoholethoxylat mit 10 Ethylenoxideinheiten (EO) und einer OHZ von 90.

Beispiel 4

4.1 Veresterung

225 g (1,1 mol) Citronensäure Monohydrat, 506 g (1,1 mol) REWOPAL[R] TA 6*und 0,7 g hypophosphorige Säure wurden unter $N_2$ in ein Reaktionsgefäß, ausgerüstet mit Rührer, Thermometer, einem Wasserabscheider und Kühler eingefüllt.

Die Mischung wurde unter einer Stickstoffatmosphäre und gutem Rühren auf 140 °C erhitzt und 4 h bei dieser Temperatur gehalten. Während dieser Zeit destillierte nahezu die theoretische Menge des Reaktionswassers ab. Man ließ die Reaktionslösung auf 90 °C abkühlen und legte ein Vakuum von 20 mbar an, um die Umsetzung zu vervollständigen. Die Säurezahl des Endprodukts lag bei 173.

4.2 Ethoxylierung

635 g Citronensäuremonoester aus Beispiel 4.1 wurden nach Zusatz von 3 g Alkyldimethylamin bei 90 °C im Autoklaven portionsweise mit 97 g Ethylenoxid umgesetzt, so daß der Druck maximal 5 bar betrug.

| Analysendaten: | |
|---|---|
| Säurezahl | 26 mg KOH/g |
| Hydroxylzahl | 138 mg KOH/g |

4.3 Halbester-Bildung

293 g (0,7 mol) Ethoxylat aus Beispiel 4.2 wurden bei 70 - 80 °C unter $N_2$ mit 59 g (0,6 mol) Maleinsäureanhydrid versetzt und 2 h bei dieser Temperatur gerührt. Das Reaktionsprodukt hatte die folgenden Analysenzahlen:

| | |
|---|---|
| Säurezahl in Aceton | 133 mg KOH/g |
| Säurezahl in Isopropanol | 122 mg KOH/g |

4.4 Sulfierung

Zu einer Lösung von 66 g (0,6 mol) Natriumsulfit in 535 g Wasser wurden bei 60 - 70 °C 293 g Halbester aus Beispiel 4.3 bei dieser Temperatur langsam zugetropft. Die Lösung wies folgende Analysendaten auf:

| | |
|---|---|
| Trockenrückstand | 39,4 % |
| Benzavlon-WAS | 28,9 % |
| pH-Wert (5 %) | 6,4 |

* = Produkt und Warenzeichen der Fa. REWO Chemische Werke GmbH; $C_{16/18}$ Fettalkoholethoxylat mit 6 Ethylenoxideinheiten (EO) und einer OHZ von 120.

Beispiel 5

5.1 Veresterung

210 g (1 mol) Citronensäure Monohydrat, 735 g (2 mol) REWOPAL[R] LA 4*und 1 g hypophosphorige Säure wurden unter $N_2$ in ein Reaktionsgefäß, ausgerüstet mit Rührer, Thermometer, einem Wasserabscheider und Kühler, eingefüllt.

Die Mischung wurde unter einer Stickstoffatmosphäre und gutem Rühren auf 140 °C erhitzt und 2 h bei dieser Temperatur gehalten. Während dieser Zeit destillierte nahezu die theoretische Menge des Reaktionswassers ab. Man ließ die Reaktionsmischung auf 80 °C abkühlen und legte ein Vakuum von 20 mbar an, um die Umsetzung zu vervollständigen. Die Säurezahl des Endprodukts lag bei 68.

5.2 Ethoxylierung

837 g Citronensäuremonoester aus Beispiel 5.1 wurden nach Zusatz von 4,1 g Alkyldimethylamin bei 90 °C im Autoklaven portionsweise mit 45 g Ethylenoxid umgesetzt, so daß der Druck maximal 5 bar betrug.

| Analysendaten: | |
|---|---|
| Säurezahl | 10,6 mg KOH/g |
| Hydroxylzahl | 92,4 mg KOH/g |

5.3 Halbester-Bildung

364 g (0,6 mol) Ethoxylat aus Beispiel 5.2 wurden bei 70 - 80 °C unter $N_2$ mit 59 g (0,6 mol) Maleinsäureanhydrid versetzt und 2 h bei dieser Temperatur gerührt. Das Reaktionsprodukt hatte die folgenden Analysenzahlen:

| | |
|---|---|
| Säurezahl in Aceton | 103 mg KOH/g |
| Säurezahl in Isopropanol | 92 mg KOH/g. |

5.4 Sulfierung

Zu einer Lösung von 66 g (0,6 mol) Natriumsulfit in 625 g Wasser wurden bei 60 - 70 °C 352 g Halbester aus Beispiel 5.3 bei dieser Temperatur langsam zugetropft. Die Lösung wies folgende Analysendaten auf:

| | |
|---|---|
| Trockenrückstand | 39,4 % |
| Benzavlon-WAS | 27,8 % |
| pH-Wert (5 %) | 6,9 |

Kosmetische Formulierungen

Die nachstehenden Formulierungen können durch einfaches Einrühren der Rezepturbestandteile in Wasser hergestellt werden.

Alle Rezepturen werden in Gewichtsprozent auf Feststoff berechnet angegeben.

* = Produkt und Warenzeichen der Fa. REWO Chemische Werke GmbH; $C_{12/14}$ Fettalkoholethoxylat mit 4 Ethylenoxideinheiten (EO) und einer OHZ von 153.

Erklärung der eingesetzten Tenside nach CTFA-Namen:

1 = Disodium PEG-4 Cocoamido MIPA-Sulfosuccinate
2 = Disodiumlauroamphodiacetate
(and) Sodium Lauryl Sulfate
(and) Hexylene Glycol
3 = Cocoamidopropyl Hydroxysultaine
4 = Ricinoleamidopropyltrimonium Methosulfate
5 = PEG-200 Glyceryl Tallowate mod.
6 = Disodium Laureth Sulfosuccinate
7 = Sodium Laureth Sulfate
8 = Cocoamidopropyl Betaine
9 = PEG-200 Glyceryl Tallowate
10 = Disodiumcocoamphodiacetate
11 = PEG-80 Glyceryl Tallowate
12 = Cocoamidopropyl Betaine
13 = Glyceryl Stearate
14 = Laureth-6

Analysenmethoden:

**Calciumhärtevertraglichkeit:** DIN 53 905
**Hautverträglichkeit (Zein-Test):**
Götte, Ernst, Chem. Phys. Appl. Surface Active Subst. Proc. Int. Congr.4 (1964) 83-90:
<200 mgN/100 ml = nicht irritierend
200-400 mgN/100 ml = leicht irritierend
>400 mgN/100 ml = irritierend
**Oberflächenspannung:**
Dr. R. Hensch; Fette, Seifen, Anstrichmittel, 72 (1970), S. 969-977
**Viskosität:** Brookfiled Rotationsviskosimeter (Becher und Spindel) gemessen bei 20 °C nach den Angaben des Geräteherstellers.

Bestimmung des **Schaumvermögens** nach Ross Miles in Anlehnung an DIN 53902 Teil 2 mit der Abänderung, daß folgende Maße und Mengen verwendet wurden:

| | |
|---|---|
| Innendurchmesser der Auslaufdüse: | 3,5 mm |
| Fallhöhe der Probelösung: | 940 mm |
| Menge der vorgelegten Probelösung: | 50 ml |
| Menge der zulaufenden Probelösung: | 200 ml |
| Meßtemperatur | 40 °C |
| Lösungsmittel | demineralisiertes Wasser |

Grundrezeptur: Hautreinigungsmittel und Make-up-Entferner für empfindliche Haut

| | |
|---|---|
| Erfindungsgemäße Verbindung | 2 - 8 Gew.-Teile |
| REWOTERIC$^{R*}$ AM B 14 [12)] | 2 - 8 Gew.-Teile |
| Hydroxyethylcellulose | 0,2 - 1,5 Gew.-Teile |
| demin. Wasser | ad 100 |

* = Warenzeichen der Fa. REWO Chemie Werke GmbH, Steinau an der Straße

Grundrezeptur: Hautcreme

| | |
|---|---|
| Erfindungsgemäße Verbindung | 1 - 5 Gew.-Teile |
| Glycerinmonodistearat | 2 - 10 Gew.-Teile |
| Cetylalkohol | 1 - 4 Gew.-Teile |
| Paraffinöl 3,5 ° E | 4 - 12 Gew.-Teile |
| Glycerin | 1 - 5 Gew.-Teile |
| demin. Wasser | ad 100 |
| Konservierungsmittel | n. B. |

Grundrezeptur: Spülmittel

| | |
|---|---|
| Erfindungsgemäße Verbindung | 1 - 10 Gew.-Teile |
| REWOPOL$^R$ *NL 3-28 [7)] | 1 - 30 Gew.-Teile |
| REWOTERIC$^R$ AM CAS [3)] | 1 - 5 Gew.-Teile |
| REWOPOL$^R$ LA 6 [14)] | 1 - 10 Gew.-Teile |
| demin. Wasser | ad 100 |

* = Warenzeichen der Fa. REWO Chemie Werke GmbH, Steinau an der Straße

Grundrezeptur: Duschbad

| | |
|---|---|
| Erfindungsgemäße Verbindung | 2 - 8 Gew.-Teile |
| REWOTERIC$^R$ AM G 30 [2)] | 5 - 15 Gew.-Teile |
| REWOTERIC$^R$ AM CAS [3)] | 2 - 6 Gew.-Teile |
| REWOQUAT$^{R*}$ RTM 50 [4)] | 1 - 3 Gew.-Teile |
| REWODERM$^{R*}$ Li S 75 [5)] | 1 - 4 Gew.-Teile |
| demin. Wasser | ad 100 |

* = Warenzeichen der Fa. REWO Chemie Werke GmbH, Steinau an der Straße

Grundrezeptur: Haarshampoo

| | |
|---|---|
| Erfindungsgemäße Verbindung | 2 - 8 Gew.-Teile |
| REWOPOL$^R$ NL 3-28 [7)] | 4 - 10 Gew.-Teile |
| REWOTERIC$^R$ AM B 13 [8)] | 2 - 8 Gew.-Teile |
| REWODERM$^R$ Li 420-70 [9)] | 1 - 4 Gew.-Teile |
| demin. Wasser | ad 100 |

Grundrezeptur: Babyshampoo

| | |
|---|---|
| Erfindungsgemäße Verbindung<br>REWOTERIC$^R$ AM 2 C NM [10)]<br>REWOTERIC$^R$ AM B 13 [8)]<br>REWOPOL$^R$ NL 3-28 [7)]<br>REWODERM$^R$ Li 48-50 [11)]<br>demin. Wasser | 2 - 8 Gew.-Teile<br>2 - 8 Gew.-Teile<br>4 - 12 Gew.-Teile<br>2 - 10 Gew.-Teile<br>1 - 4 Gew.-Teile<br>ad 100 |

Tabelle 1

| Verbindung gemäß Beispiel | a + b | c | x• $R^1$ x = | y• $R^2$ = H z• $R^2$ = $R^1$ m• $R^2$ = $R^3$ | $R^3$ = Cn n = |
|---|---|---|---|---|---|
| 1.4 | 2 | 4 | 1,0 | y 0,4<br>z 0,6<br>m - | 12/14 |
| 2.4 | 2,7 | 6 | " | y 0,4<br>z 0,6<br>m - | 12/14 |
| 3.4 | 2,7 | 10 | " | y 0,4<br>z 0,6<br>m - | 12/14 |
| 4.4 | 2,7 | 6 | " | y 0,4<br>z 0,6<br>m - | 18 |
| 5.4 | 5 | 4 | " | y 0<br>z 0<br>m 1 | 12/14 |

Tabelle 2

| Beispiel aus Tabelle 1 | Schaumvermögen [mm] sofort | nach 5 Min. | Calciumhärteverträglichkeit Punkte | Einstufung | Zeintest mgN/100ml | Oberflächenspannung Lecomte du Nouy (Ringmethode) mN/m 0,1 % | 0,01 % |
|---|---|---|---|---|---|---|---|
| Beispiel 1.4 | 150 | 140 | 75 | V | 25 | 33,7 | 35,7 |
| Beispiel 2.4 | 155 | 150 | 75 | V | 30 | 33,3 | 35,2 |
| Beispiel 3.4 | 145 | 140 | 75 | V | 12 | 37,7 | 37,9 |
| Beispiel 4.4 | 100 | 70 | 75 | V | 41 | 43,5 | 44,8 |
| Beispiel 5.4 | 140 | 130 | 75 | V | | 32,0 | 34,9 |
| Vergleichsbeispiel REWOPOL® NL 3-28 | 200 | 180 | 75 | V | 300 | 34,2 | 32,9 |
| Vergleichsbeispiel REWOPOL® SB FA 30 | 170 | 160 | 75 | V | 250 | 28,0 | 31,4 |

Testrezeptur: Duschbad

Die erfindungsgemäßen Beispiele sind in der Testrezeptur von einem Testpanel von 20 Personen (weiblich und männlich) bewertet worden bezüglich

- Anschäumvermögen

- Hautgefühl
- Hautgefühl der abgetrockneten Haut

Hautverträglichkeit: Reduzierung der Zeinwerte in Rezeptur Tabelle 4.

Tabelle 3

| Rezeptur | 1 | 2 | 3 |
|---|---|---|---|
| Beispiel 1.4 | - | 4 | - |
| Beispiel 3.4 | - | - | 4 |
| REWOPOL$^R$ SB Z [1)] | 4 | - | - |
| REWOTERIC$^R$ AM G 30 [2)] | 10,5 | 10,5 | 10,5 |
| REWOTERIC$^R$ AM CAS [3)] | 3 | 3 | 3 |
| REWOQUAT$^R$ RTM 50 [4)] | 1 | 1 | 1 |
| REWODERM$^R$ Li S 75 [5)] | 2,3 | 2,3 | 2,3 |
| demin. Wasser | ad 100 | ad 100 | ad 100 |
| pH-Wert, mit Citronensäure eingestellt | 6,5 | 6,5 | 6,5 |
| Viskosität [mPas] | 3000 | 3000 | ~ 2500 |
| Schaumvermögen [mm] | 195/185 | 205/195 | 200/195 |
| Anschäumvermögen[A)] | gut | gut | gut |
| Schaumstruktur [A)] | feinblasig | cremig, feinblasig | cremig, feinblasig |
| Hautgefühl, nasse Haut [A)] | relativ glatt | glatt | glatt |
| Hautgefühl, trockene Haut [A)] | relativ glatt | glatt | glatt |

[A)] = Die Bewertung erfolgte nach einem abgestuften Bewertungssystem, wobei die oben angeführte Beurteilung den arithmetischen Mittelwert wiedergibt

Tabelle 4

Testrezeptur: Hautreinigungspräparat, Reduzierung Zein-Werte

| | 1 | 2 | 3 | 4 |
|---|---|---|---|---|
| Beispiel 3.4 | - | 4,5 | - | - |
| Beispiel 1.4 | 4,5 | - | - | - |
| REWOPOL® SB FA 30 [6)] | - | - | 4,5 | - |
| REWOPOL® NL 3-28 [7)] | 10,5 | 10,5 | 10,5 | 15 |
| demin. Wasser | 85 | 85 | 85 | 85 |
| pH-Wert, mit Citronensäure eingestellt | 6,5 | 6,5 | 6,5 | 6,5 |
| Bewertung der Schaumqualität | cremig, feinblasig | cremig, feinblasig | feinblasig | grobblasig |
| Hautgefühl nach dem Waschen auf der trockenen Haut | angenehm glatt | angenehm glatt | weich | etwas rauh |
| Zein-Werte Hautverträglichkeit | 180 mgN/100 ml | 180 mgN/100 ml | 270 mgN/100 ml | 300 mgN/100 ml |

Testrezeptur: Haarshampoo

Die erfindungsgemäßen Beispiele sind in der Rezeptur von 10 in der Anwendung erfahrenen Testpersonen auf

- Kämmbarkeit

- Volumen des Haares und Sitz der Frisur

bewertet worden.

Tabelle 5

| Rezeptur | 1 | 2 | 3 |
|---|---|---|---|
| Beispiel 1.4<br>Beispiel 2.4<br>REWOPOL[R] NL 3-28 [7)]<br>REWOTERIC[R] AM B 13 [8)]<br>REWODERM[R] Li 420-70 [9)]<br>demin. Wasser | -<br>-<br>8<br>5<br>2,1<br>ad 100 | 3<br>-<br>6<br>4<br>2,1<br>ad 100 | -<br>3<br>6<br>4<br>2,1<br>ad 100 |
| pH-Wert, mit Citronensäure eingestellt | 6,5 | 6,5 | 6,5 |
| Kämmbarkeit | 3 | 5-6 | 5-6 |
| Volumen des Haares Sitz der Frisur | 3-4 | 6 | 6 |
| Viskosität [mPas]<br>Schaumhöhe [mm] | ~3000<br>195/185 | ~12000<br>195/185 | ~12000<br>195/185 |

Bewertung der Kämmbarkeit:

1 - 3: schlecht durchzukämmen, das Haar setzt dem Kämmvorgang erheblichen Widerstand entgegen

4 - 7: das Haar läßt sich relativ gut durchkämmen, Kämmwiderstand verringert, je nach Haartyp ausreichend für ein Conditioning-Shampoo

8 -10: vorbehalten für die nachfolgende Nachbehandlung durch sogenannte Hair-Rinse-Mittel

Bewertung des Haarvolumens und des Sitzes der Frisur

1 - 3: das Haar ist trocken oder strohig, schlechter Sitz der Frisur

4 - 7: das Haar ist weich und füllig, bei gleichzeitig gutem Sitz der Frisur

8 -10: das Haar ist weich und glatt, jedoch zu locker, dadurch kein guter Sitz der Frisur.

Die Bewertung erfolgte nach einem abgestuften Punktsystem, wobei die oben angegebene Beurteilung den arithmetischen Mittelwert wiedergibt.

Testrezeptur: Babyshampoo bzw. Haar- und Körpershampoo für empfindliche Haut

Tabelle 6

| Rezeptur | 1 | 2 | 3 |
|---|---|---|---|
| Beispiel 1.4<br>Beispiel 2.4<br>REWOTERIC$^R$ AM 2C NM 10)<br>REWOTERIC$^{R)}$ AM B 13 8)<br>REWOPOL$^{R)}$ NL 3-28 7)<br>REWODERM$^{R)}$ Li 48-50 11)<br>demin. Wasser | -<br>-<br>3,5<br>7<br>7,5<br>3<br>ad 100 | 3<br>-<br>3,5<br>7<br>4,5<br>3<br>ad 100 | -<br>3<br>3,5<br>7<br>4,5<br>3<br>ad 100 |
| pH-Wert, mit Citronensäure eingestellt | 6,5 | 6,5 | 6,5 |
| Schaumhöhe mm DIN 53 902 Teil 2 | 195/185 | 200/190 | 200/190 |
| Zeinwerte, mgN/100 ml (Hautverträglichkeit) | ˜70 | ˜70 | ˜70 |
| Schaumstruktur | grobblasig | feinblasig | feinblasig |
| Hautgefühl, nasse und trockene Haut | etwas rauh | glatt weich | glatt weich |

Testrezeptur: Hautreinigungsmittel und Make-up-Entferner für empfindliche Haut

Tabelle 7

| Rezeptur | 1 | 2 | 3 |
|---|---|---|---|
| Beispiel 3.4<br>Beispiel 1.4<br>REWOPOL$^R$ SB FA 30 6)<br>REWOTERIC AM B 14 12)<br>Hydroxyethylcellulose<br>demin. Wasser | -<br>-<br>4<br>4,5<br>0,5<br>ad 100 | -<br>4<br>-<br>4,5<br>0,5<br>ad 100 | 4<br>-<br>-<br>4,5<br>0,5<br>ad 100 |
| pH-Wert, mit Citronensäure eingestellt | 6,5 | 6,5 | 6,5 |
| Zein-Werte mgN/100 ml (Hautverträglichkeit) | ca. 170 | <50 | <50 |

Der Einsatz der erfindungsgemäßen Sulfosuccinate in der vorliegenden Rezeptur ergibt ein sehr gut hautverträgliches Reinigungsmittel, was sich im niedrigen Zein-Wert zeigt im Vergleich zum Standard Sulfosuccinate REWOPOL$^R$ SB FA 30.

Tabelle 8 Testrezeptur Hautcreme

| Rezeptur | 1 | 2 | 3 | 4 |
|---|---|---|---|---|
| Beispiel 1.4 | - | - | 2 | - |
| Beispiel 3.4 | - | - | - | 2 |
| Stearylalkohol-ethoxylat (3EO) | 2 | - | - | - |
| REWOMUL® MG [13] | 6 | 6 | 6 | 6 |
| REWOPOL® SB FA 30 [6] | - | 2 | - | - |
| Cetylalkohol | 2 | 2 | 2 | 2 |
| Paraffinöl 3,5°E | 8 | 8 | 8 | 8 |
| Glycerin | 3 | 3 | 3 | 3 |
| Konservierungsmittel | n.B. | n.B. | n.B. | n.B. |
| demin. Wasser | ad 100 | ad 100 | ad 100 | ad 100 |
| Stabilität der Emulsion bei 5 °C, 20 °C und 40 °C | stabil | instabil | stabil | stabil |
| Aussehen | glatte, weiße Creme | griesig, Emulsion bricht | glatte, weiße Creme | glatte, weiße Creme |

Bewertung der Eigenschaften auf der Haut durch 10 Testpersonen; mehr als 80 % beurteilten die Testrezepturen 3 und 4 als

- gut auf der Haut zu verstreichen
- gut in die Haut einziehend
- die Haut wird glatt und weich, ohne klebrig, fettig zu sein.

Die Vergleichsrezeptur 1 wird gleich bewertet, die Rezeptur 2 ist instabil.

Tabelle 9 Testrezeptur: Hautfreundliche Geschirrspülmittel zur manuellen Anwendung

| Rezeptur | 1 | 2 | 3 | 4 | 5 | 6 |
|---|---|---|---|---|---|---|
| Beispiel 3.4 | - | - | 3 | - | 1,5 | - |
| Beispiel 1.4 | - | 3 | - | 1,5 | - | - |
| REWOPOL® NL 3-28 [7)] | 18 | 18 | 18 | 18 | 18 | 18 |
| REWOTERIC® AM CAS [3)] | 1,5 | - | - | 1,5 | 1,5 | - |
| REWOPOL® LA 6 [4)] | 1,5 | - | - | - | 1,5 | 3 |
| demin. Wasser | ad 100 | ad 100 | ad 100 | ad 100 | ad 100 | ad 100 |
| Viskosität m Pa.s mit NaCl eingestellt auf ca. | 500 | 500 | 500 | 500 | 500 | 500 |
| Zein-Werte (Hautverträglichkeit) | 220 | 220 | 180 | 180 | 200 | 250 |
| Schaumvermögen [mm] | 195/185 | 195/185 | 190/185 | 190/185 | 190/185 | 190/180 |
| + 0,5 ml Olivenöl | 190/180 | 190/185 | 190/185 | 190/180 | 190/185 | 190/180 |
| + 1,0 ml Olivenöl | 190/185 | 190/185 | 190/185 | 185/180 | 190/185 | 190/180 |

**Patentansprüche**

**Patentansprüche für folgende Vertragsstaaten : BE, DK, DE, GB, FR, GR, NL, IT, LU, AT, SE, CH, LI**

1. Citronensäurefettalkoholesterpolyglykolethersulfosuccinate der allgemeinen Formel (1)

$$\begin{array}{l} H_2C-COO-(CHR-CH_2-O)_a-R^1 \\ \quad\;\; | \\ HO-C-COO(CHR-CH_2-O)_b-R^2 \\ \quad\;\; | \\ H_2C-COO(CHR-CH_2-O)_c-R^3 \end{array} \qquad (1)$$

worin

R = gleich oder verschieden H oder $-CH_3$

$R^1$ =

$$\begin{array}{l} -CO-CH-CH_2-COO^-X^+ \\ \quad\quad\;\; | \\ \quad\quad SO_3^-X^+ \end{array} \quad \text{oder} \quad \begin{array}{l} -CO-CH_2-CH-COO^-X^+ \\ \quad\quad\quad\quad\quad\;\; | \\ \quad\quad\quad\quad\quad SO_3^-X^+ \end{array}$$

$R^2$ = $R^1$ oder H oder $R^3$

$R^3$ = ein gegehenenfalls substituierter und/oder Mehrfachbindungen enthaltender Alkyl- oder Acylrest mit 8 - 22 C-Atomen

a,b = gleich oder verschieden 0 - 5 sein kann, wobei $a+b \geq 1$ liegt und

c = 0 - 15 und

$x^+$ = $H^+$ oder ein Kation ist.

2. Verbindungen der allgemeinen Formel (1) gemäß Anspruch 1, dadurch gekennzeichnet, daß

R = H

$R^1$, $R^2$ =

$$\begin{array}{l} -CO-CH-CH_2-COONa \\ \quad\quad\;\; | \\ \quad\quad SO_3^-Na^+ \end{array} \quad \text{oder} \quad \begin{array}{l} -CH-CH_2-CH-COONa- \\ \quad\quad\quad\quad\quad\; | \\ \quad\quad\quad\quad SO_3^-Na^+ \end{array}$$

$R^3$ = Alkylrest mit 12 - 18 C-Atomen

a + b = 2 - 5

c = 1 - 10 und

$x^+$ = ein Kation ist.

3. Verbindungen der allgemeinen Formel (1) gemäß Anspruch 2, dadurch gekennzeichnet, daß a + b + c = 5 - 15, $R^3$ = Acylrest mit 8 - 18 C-Atomen aus der natürlichen Mischung der Kokosfettsäuren ist.

4. Verfahren zur Herstellung der Verbindungen der allgemeinen Formel (1)

$$\begin{array}{l} H_2C-COO-(CHR-CH_2-O)_a-R^1 \\ \quad\;\; | \\ HO-C-COO(CHR-CH_2-O)_b-R^2 \\ \quad\;\; | \\ H_2C-COO(CHR-CH_2-O)_c-R^3 \end{array} \qquad (1)$$

worin

R = gleich oder verschieden H oder $-CH_3$

$R^1$ =

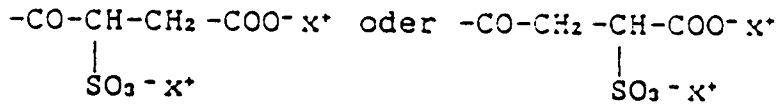

$R^2$ = $R^1$ oder H oder $R^3$

$R^3$ = ein gegebenenfalls substituierter und/oder Mehrfachbindungen enthaltender Alkyl- oder Acylrest mit 8 - 22 C-Atomen

a,b = gleich oder verschieden 0 - 5 sein kann, wobei $a+b \geqq 1$ liegt und

c = 0 - 15 und

$x^+$ = $H^+$ oder ein Kation ist,

dadurch gekennzeichnet, daß in erster Stufe die Citronensäuremonoester mit dem Fettalkohol bzw. dem Fettalkoholalkoxylat, gegebenenfalls unter Mitverwendung eines Veresterungskatalysators, bei 140 - 160 °C unter Entfernung des Reaktionswassers verestert, dieser in zweiter Stufe in Gegenwart eines basischen Katalysators bei 80 - 150 °C und 1 - 10 bar mit Alkylenoxid zum Alkoxilat umgesetzt wird, welches in dritter Stufe durch Addition von Maleinsäureanhydrid bei 60 - 80 °C zum Halbester und in letzter Stufe bei 60 - 80 °C mit wässriger Natriumsulfitlösung zum Salz der entsprechenden Sulfonsäure umgesetzt wird.

**5.** Verfahren gemäß Anspruch 4, dadurch gekennzeichnet, daß in erster Stufe die Alkoxylate und in zweiter Stufe die Ester hergestellt werden.

**6.** Wässrige Haarreinigungs- und Pflegemittel, enthaltend
a) 1 - 10 Gew.-Teile mindestens eine der Verbindungen der allgemeinen Formel (1) und
b) 1 - 20 Gew.-Teile mindestens eines Tensides aus der Gruppe der nichtionischen amphoteren, zwitterionischen, ionischen Tenside und gegebenenfalls
c) 0,1 - 10 Gew.-Teile Verdickungsmittel, Duftstoffe, Konservierungsstoffe, Farbstoffe, Pflanzenextrakte und sonstige Zusatz- und Hilfsstoffe und
d) ad 100 Wasser.

**7.** Wässriges Dusch- und Haarshampoo, enthaltend
a) 1- 10 Gew.-Teile der Verbindungen der allgemeinen Formel (1)
b) 1 - 20 Gew.-Teile mindestens eines nichtionischen, amphoteren, zwitterionischen, ionischen Tensids und
c) 0,1 - 10 Gew.-Teile Verdickungsmittel, Duftstoffe, Konservierungsmittel, Farbstoffe, Pflanzenextrakte und sonstige Zusatz- und Hilfsstoffe und gegebenenfalls
d) ad 100 Wasser.

**8.** Wässriges Geschirrspülmittel, enthaltend
a) 1 - 10 Gew.-Teile mindestens einer der Verbindungen der allgemeinen Formel (1) und
b) 1 - 30 Gew.-Teile mindestens eines Tensids aus der Gruppe der nichtionischen, amphoteren, zwitterionischen, ionischen Tenside und gegebenenfalls
c) 0,1 - 10 Gew.-Teile Verdickungsmittel, Duftstoffe, Konservierungsmittel, Farbstoffe, Pflanzenextrakte und sonstige Zusatz- und Hilfsstoffe und gegebenenfalls
d) ad 100 Wasser.

**9.** Hautpflegemittel, enthaltend
a) 1 - 10 Gew.-Teile mindestens einer der Verbindungen der allgemeinen Formel (1) und
b) 1 - 10 Gew.-Teile mindestens eines Tensids aus der Gruppe der nichtionischen, amphoteren, zwitterionischen, ionischen Tenside und gegebenenfalls
c) 2 - 10 Gew.-Teile pflanzliche oder mineralische Oele, Esteroele
d) 1 - 5 Gew.-Teile Konsistenzgeber
e) 0,5 - 5,0 Gew.-Teile Duftstoffe, Farbstoffe, Konservierungsmittel
f) ad 100 Wasser.

**10.** Hautreinigungsmittel, enthaltend
a) 1 - 10 Gew.-Teile mindestens einer der Verbindungen der allgemeinen Formel (1) und

b) 0,1 - 20 Gew.-Teile mindestens eines Tensids aus der Gruppe der nichtionischen, amphoteren, zwitterionischen, ionischen Tenside und gegebenenfalls

c) 0,1 - 10 Gew.-Teile Verdickungsmittel, Duftstoffe, Konservierungsmittel, Farbstoffe, Pflanzenextrakte und sonstige Zusatz- und Hilfsstoffe und gegebenenfalls

d) ad 100 Wasser.

**Patentansprüche für folgenden Vertragsstaat : ES**

**1.** Verfahren zur Herstellung der Verbindungen der allgemeinen Formel (1)

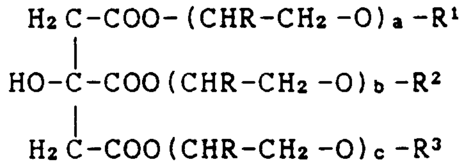

(1)

worin

R = gleich oder verschieden H oder $-CH_3$

$R^1$ =

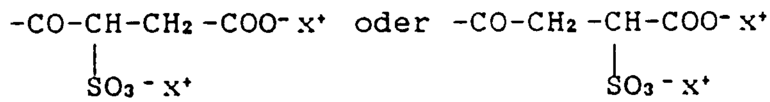

$R^2$ = $R^1$ oder H oder $R^3$

$R^3$ = ein gegebenenfalls substituierter und/oder Mehrfachbindungen enthaltender Alkyl- oder Acylrest mit 8 - 22 C-Atomen

a,b = gleich oder verschieden 0 - 5 sein kann, wobei $a+b \geq 1$ liegt und

c = 0 - 15 und

$x^+$ = $H^+$ oder ein Kation ist,

dadurch gekennzeichnet, daß in erster Stufe die Citronensäuremonoester mit dem Fettalkohol bzw. dem Fettalkoholalkoxylat, gegebenenfalls unter Mitverwendung eines Veresterungskatalysators, bei 140 - 160 °C unter Entfernung des Reaktionswassers verestert, dieser in zweiter Stufe in Gegenwart eines basischen Katalysators bei 80 - 150 °C und 1 - 10 bar mit Alkylenoxid zum Alkoxilat umgesetzt wird, welches in dritter Stufe durch Addition von Maleinsäureanhydrid bei 60 - 80 °C zum Halbester und in letzter Stufe bei 60 - 80 °C mit wässriger Natriumsulfitlösung zum Salz der entsprechenden Sulfonsäure umgesetzt wird.

**2.** Verfahren zur Herstellung von Verbindungen der allgemeinen Formel (1) gemäß Anspruch 1, dadurch gekennzeichnet, daß

R = H

$R^1$, $R^2$ =

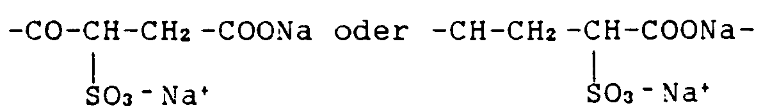

$R^3$ = Alkylrest mit 12 - 18 C-Atomen

$a+b$ = 2 - 5

c = 1 - 10 und

$x^+$ = ein Kation ist.

**3.** Verfahren zur Herstellung von Verbindungen der allgemeinen Formel (1) gemäß Anspruch 1, dadurch gekennzeichnet, daß $a+b+c$ = 5 - 15, $R^3$ = Acylrest mit 8 - 18 C-Atomen aus der natürlichen Mischung der Kokosfettsäuren ist.

**4.** Verfahren gemäß den Ansprüchen 1 - 3, dadurch gekennzeichnet, daß in erster Stufe die Alkoxylate und in zweiter Stufe die Ester hergestellt werden.

**5.** Wässrige Haarreinigungs- und Pflegemittel, enthaltend
a) 1 - 10 Gew.-Teile mindestens eine der Verbindungen der allgemeinen Formel (1) und
b) 1 - 20 Gew.-Teile mindestens eines Tensides aus der Gruppe der nichtionischen amphoteren, zwitterionischen, ionischen Tenside und gegebenenfalls
c) 0,1 - 10 Gew.-Teile Verdickungsmittel, Duftstoffe, Konservierungsstoffe, Farbstoffe, Pflanzenextrakte und sonstige Zusatz- und Hilfsstoffe und
d) ad 100 Wasser.

**6.** Wässriges Dusch- und Haarshampoo, enthaltend
a) 1- 10 Gew.-Teile der Verbindungen der allgemeinen Formel (1)
b) 1 - 20 Gew.-Teile mindestens eines nichtionischen, amphoteren, zwitterionischen, ionischen Tensids und
c) 0,1 - 10 Gew.-Teile Verdickungsmittel, Duftstoffe, Konservierungsmittel, Farbstoffe, Pflanzenextrakte und sonstige Zusatz- und Hilfsstoffe und gegebenenfalls
d) ad 100 Wasser.

**7.** Wässriges Geschirrspülmittel, enthaltend
a) 1 - 10 Gew.-Teile mindestens einer der Verbindungen der allgemeinen Formel (1) und
b) 1 - 30 Gew.-Teile mindestens eines Tensids aus der Gruppe der nichtionischen, amphoteren, zwitterionischen, ionischen Tenside und gegebenenfalls
c) 0,1 - 10 Gew.-Teile Verdickungsmittel, Duftstoffe, Konservierungsmittel, Farbstoffe, Pflanzenextrakte und sonstige Zusatz- und Hilfsstoffe und gegebenenfalls
d) ad 100 Wasser.

**8.** Hautpflegemittel, enthaltend
a) 1 - 10 Gew.-Teile mindestens einer der Verbindungen der allgemeinen Formel (1) und
b) 1 - 10 Gew.-Teile mindestens eines Tensids aus der Gruppe der nichtionischen, amphoteren, zwitterionischen, ionischen Tenside und gegebenenfalls
c) 2 - 10 Gew.-Teile pflanzliche oder mineralische Oele, Esteroele
d) 1 - 5 Gew.-Teile Konsistenzgeber
e) 0,5 - 5,0 Gew.-Teile Duftstoffe, Farbstoffe, Konservierungsmittel
f) ad 100 Wasser.

**9.** Hautreinigungsmittel, enthaltend
a) 1 - 10 Gew.-Teile mindestens einer der Verbindungen der allgemeinen Formel (1) und
b) 0,1 - 20 Gew.-Teile mindestens eines Tensids aus der Gruppe der nichtionischen, amphoteren, zwitterionischen, ionischen Tenside und gegebenenfalls
c) 0,1 - 10 Gew.-Teile Verdickungsmittel, Duftstoffe, Konservierungsmittel, Farbstoffe, Pflanzenextrakte und sonstige Zusatz- und Hilfsstoffe und gegebenenfalls
d) ad 100 Wasser.

## Claims

**Claims for the following Contracting States : BE, DK, DE, GB, FR, GR, NL, IT, LU, AT, SE, CH, LI**

**1.** Citric acid fatty alcohol ester polyglycol ether sulphosuccinates of the general formula (1)

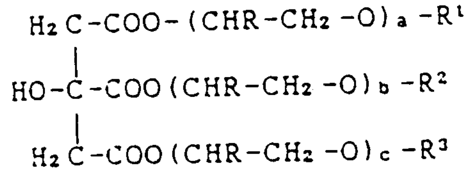

(1)

in which
each R is the same or different and represents H or $-CH_3$

$R^1$ =

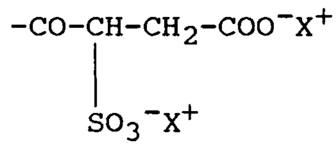 or 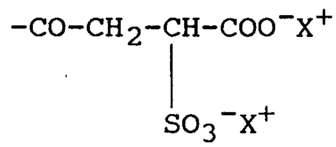

$R_2$ = $R^1$ or H or $R^3$
$R^3$ = an alkyl or acyl radical having 8-22 carbon atoms, which is optionally substituted and/or optionally contains multiple bonds,
a and b may be the same or different and are each 0-5, wherein $a+b \geqq 1$ and
c = 0-15 and
$X^+$ = $H^+$ or a cation.

**2.** Compounds of the general formula (1) according to claim 1, characterised in that
R = H,
$R^1$, $R^2$ =

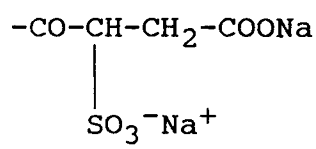 or 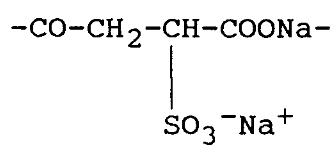

$R^3$ = an alkyl radical having 12-18 carbon atoms
a+b = 2-5
c = 1-10 and
$X^+$ = a cation.

**3.** Compounds of the general formula (1) according to claim 2, characterised in that a+b+c = 5-15, $R^3$ = an acyl radical having 8-18 carbon atoms from the natural mixture of the coconut fatty acids.

**4.** Process for the preparation of compounds of the general formula (1)

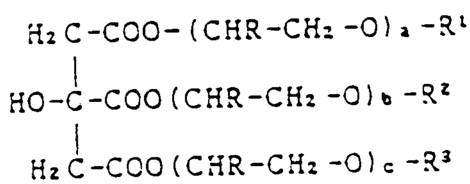 (1)

in which
each R is the same or different and represents H or $-CH_3$
$R^1$ =

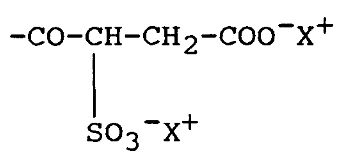 or 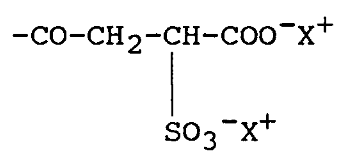

$R_2$ = $R^1$ or H or $R^3$
$R^3$ = an alkyl or acyl radical having 8-22 carbon atoms, which is optionally substituted and/or optionally contains multiple bonds,
a and b may be the same or different and are each 0-5, wherein $a+b \geqq 1$ and
c = 0-15 and
$X^+$ = $H^+$ or a cation,
characterised in that, in a first step the citric acid monoester is esterified with the fatty alcohol or the fatty alcohol alkoxylate at 140-160 °C, optionally with the co-use of an esterification catalyst, with

removal of the water of reaction, the product is reacted in a second step, at 80-150 °C and 1-10 bar in the presence of a basic catalyst, with alkylene oxide to form the alkoxylate, which in a third step is reacted by the addition of maleic acid anhydride at 60-80 °C to form the semiester and, in a last step, is reacted at 60-80 °C with aqueous sodium sulphite solution to form the salt of the corresponding sulphonic acid.

**5.** Process according to claim 4, characterised in that the alkoxylates are produced in a first step and the esters are produced in a second step.

**6.** Aqueous hair-cleaning and care agents, comprising
a) 1 - 10 parts by weight of at least one of the compounds of the general formula (1) and
b) 1 - 20 parts by weight of at least one surfactant from the group consisting of non-ionic, amphoteric, zwitterionic and ionic surfactants and, optionally,
c) 0.1 - 10 parts by weight of thickening agents, fragrances, preservatives, colourants, plant extracts and other additives and auxiliaries and
d) water to 100.

**7.** Aqueous shower and hair shampoo, comprising
a) 1 - 10 parts by weight of the compounds of the general formula (1) and
b) 1 - 20 parts by weight of at least one non-ionic, amphoteric, zwitterionic or ionic surfactant and
c) 0.1 - 10 parts by weight of thickening agents, fragrances, preservatives, colourants, plant extracts and other additives and auxiliaries and, optionally,
d) water to 100.

**8.** Aqueous washing-up detergent, comprising
a) 1 - 10 parts by weight of at least one of the compounds of the general formula (1) and
b) 1 - 30 parts by weight of at least one surfactant from the group consisting of non-ionic, amphoteric, zwitterionic and ionic surfactants and, optionally,
c) 0.1 - 10 parts by weight of thickening agents, fragrances, preservatives, colourants, plant extracts and other additives and auxiliaries and, optionally,
d) water to 100.

**9.** Skin-care agent, comprising
a) 1 - 10 parts by weight of at least one of the compounds of the general formula (1) and
b) 1 - 10 parts by weight of at least one surfactant from the group consisting of non-ionic, amphoteric, zwitterionic and ionic surfactants and, optionally,
c) 2 - 10 parts by weight of plant or mineral oils or ester oils,
d) 1 - 5 parts by weight of a consistency-imparting agent
e) 0.5 - 5.0 parts by weight of fragrances, colourants and preservatives,
f) water ad 100.

**10.** Skin-cleansing agent, comprising
a) 1 - 10 parts by weight of at least one of the compounds of the general formula (1) and
b) 0.1 - 20 parts by weight of at least one surfactant from the group consisting of non-ionic, amphoteric, zwitterionic and ionic surfactants and, optionally,
c) 0.1 - 10 parts by weight of thickening agents, fragrances, preservatives, colourants, plant extracts and other additives and auxiliaries and
d) water to 100.

**Claims for the following Contracting State : ES**

**1.** Process for the preparation of compounds of the general formula (1)

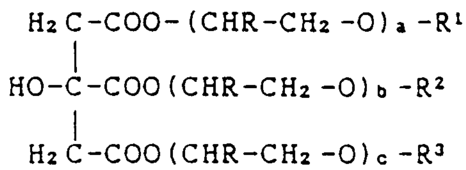

(1)

in which
each R is the same or different and represents H or $-CH_3$
$R^1$ =

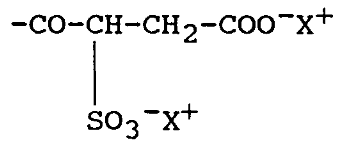

or

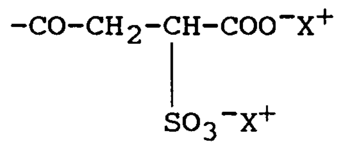

| | |
|---|---|
| $R_2$ = | $R^1$ or H or $R^3$ |
| $R^3$ = | an alkyl or acyl radical having 8-22 carbon atoms, which is optionally substituted and/or optionally contains multiple bonds, |
| a and b | may be the same or different and are each 0-5, wherein $a + b \geqq 1$ and |
| c = | 0-15 and |
| $X^+$ = | $H^+$ or a cation, |

characterised in that, in a first step the citric acid monoester is esterified with the fatty alcohol or the fatty alcohol alkoxylate at 140-160 °C, optionally with the co-use of an esterification catalyst, with removal of the water of reaction, the product is reacted in a second step, at 80-150 °C and 1-10 bar in the presence of a basic catalyst, with alkylene oxide to form the alkoxylate, which in a third step is reacted by the addition of maleic acid anhydride at 60-80 °C to form the semiester and, in a last step, is reacted at 60-80 °C with aqueous sodium sulphite solution to form the salt of the corresponding sulphonic acid.

2. Process for the preparation of compounds of the general formula (1) according to claim 1, characterised in that
R = H,
$R^1$, $R^2$ =

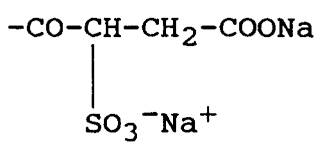

or

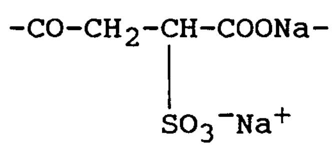

| | |
|---|---|
| $R^3$ = | an alkyl radical having 12-18 carbon atoms |
| a + b = | 2-5 |
| c = | 1-10 and |
| $X^+$ = | a cation. |

3. A process for the preparation of compounds of the general formula (1) according to claim 1, characterised in that a + b + c = 5-15, $R^3$ = an acyl radical having 8-18 carbon atoms from the natural mixture of the coconut fatty acids.

4. A process according to any one of claims 1 to 3, characterised in that the alkoxylates are produced in a first step and the esters are produced in a second step.

5. Aqueous hair-cleaning and care agents, comprising
a) 1 - 10 parts by weight of at least one of the compounds of the general formula (1) and
b) 1 - 20 parts by weight of at least one surfactant from the group consisting of non-ionic, amphoteric, zwitterionic and ionic surfactants and, optionally,

c) 0.1 - 10 parts by weight of thickening agents, fragrances, preservatives, colourants, plant extracts and other additives and auxiliaries and
d) water to 100.

**6.** Aqueous shower and hair shampoo, comprising
a) 1 - 10 parts by weight of the compounds of the general formula (1) and
b) 1 - 20 parts by weight of at least one non-ionic, amphoteric, zwitterionic or ionic surfactant and
c) 0.1 - 10 parts by weight of thickening agents, fragrances, preservatives, colourants, plant extracts and other additives and auxiliaries and, optionally,
d) water to 100.

**7.** Aqueous washing-up detergent, comprising
a) 1 - 10 parts by weight of at least one of the compounds of the general formula (1) and
b) 1 - 30 parts by weight of at least one surfactant from the group consisting of non-ionic, amphoteric, zwitterionic and ionic surfactants and, optionally,
c) 0.1 - 10 parts by weight of thickening agents, fragrances, preservatives, colourants, plant extracts and other additives and auxiliaries and, optionally,
d) water to 100.

**8.** Skin-care agent, comprising
a) 1 - 10 parts by weight of at least one of the compounds of the general formula (1) and
b) 1 - 10 parts by weight of at least one surfactant from the group consisting of non-ionic, amphoteric, zwitterionic and ionic surfactants and, optionally,
c) 2 - 10 parts by weight of plant or mineral oils or ester oils,
d) 1 - 5 parts by weight of a consistency-imparting agent
e) 0.5 - 5.0 parts by weight of fragrances, colourants and preservatives,
f) water ad 100.

**9.** Skin-cleansing agent, comprising
a) 1 - 10 parts by weight of at least one of the compounds of the general formula (1) and
b) 0.1 - 20 parts by weight of at least one surfactant from the group consisting of non-ionic, amphoteric, zwitterionic and ionic surfactants and, optionally,
c) 0.1 - 10 parts by weight of thickening agents, fragrances, preservatives, colourants, plant extracts and other additives and auxiliaries and
d) water to 100.

**Revendications**
**Revendications pour les Etats contractants suivants : BE, DK, DE, GB, FR, GR, NL, IT, LU, AT, SE, CH, LI**

**1.** Sulfosuccinates d'esters de l'acide citrique et d'alcools gras polyalcoxylés de formule générale (1)

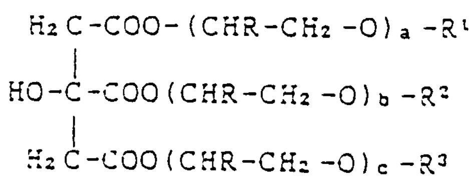

(1)

dans laquelle :
les radicaux R sont identiques ou différents et représentent chacun H ou $-CH_3$
$R^1$ =

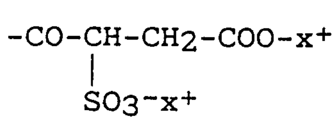
ou
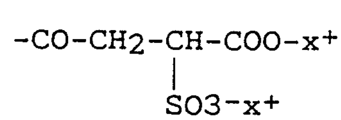

$R^2$ = $R^1$, ou H, ou $R^3$

$R^3$ est un radical alkyle ou acyle, éventuellement substitué et/ou contenant des doubles liaisons et ayant de 8 à 22 atomes de carbone,
a et b sont identiques ou différents et valent chacun de 0 à 5, avec a + b ≧ 1, et
c = 0 à 15, et
$x^+$ est $H^+$ ou est un cation.

**2.** Composés de formule générale (1) selon la revendication 1, caractérisés en ce que :
R = H,
$R^1$, $R^2$ =

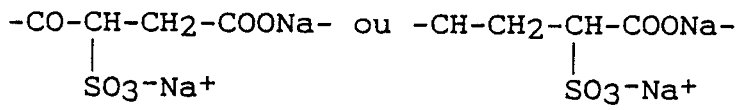

$R^3$ est un radical alkyle ayant de 12 à 18 atomes de carbone
a + b = 2 à 5
c = 1 à 10 et
$x^+$ est un cation.

**3.** Composés de formule générale (1) selon la revendication 2, caractérisés en ce que a + b + c = 5 à 15, $R^3$ est un radical acyle ayant de 8 à 18 atomes de carbone, dérivé d'un mélange naturel d'acides gras dérivés de l'huile de coprah.

**4.** Procédé pour préparer les composés de formule générale (1)

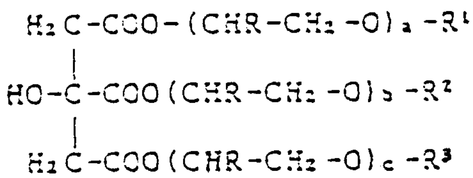

(1)

dans laquelle :
les radicaux R sont identiques ou différents et représentent chacun H ou $-CH_3$
$R^1$ =

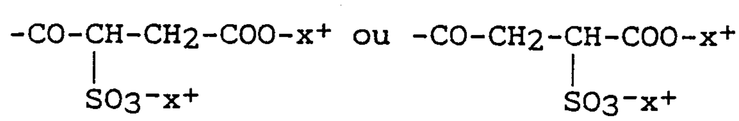

$R^2$ = $R^1$, ou H, ou $R^3$
$R^3$ est un radical alkyle ou acyle, éventuellement substitué et/ou contenant des doubles liaisons et ayant de 8 à 22 atomes de carbone,
a et b sont identiques ou différents et valent chacun de 0 à 5, avec a + b ≧ 1, et
c = 0 à 15, et
$x^+$ est $H^+$ ou est un cation,
caractérisé en ce que dans une première étape, on estérifie le monoester de l'acide citrique avec l'alcool gras ou l'alcool gras alcoxylé, en utilisant éventuellement un catalyseur d'estérification, à une température de 140 à 160 °C tout en éliminant l'eau de réaction ; dans une deuxième étape, on fait réagir ce dernier en présence d'un catalyseur basique à une température de 80 à 150 °C et sous une pression de 1 à 10 bar avec un oxyde d'alkylène pour donner un produit d'alcoxylation, lequel, dans une troisième étape, est mis à réagir, par addition d'anhydride maléique à une température de 60 à 80 °C pour donner le semi-ester, et, dans une dernière étape, à une température de 60 à 80 °C, avec une solution aqueuse de sulfite de sodium pour donner le sel de l'acide sulfonique correspondant.

**5.** Procédé selon la revendication 4, caractérisé en ce qu'on prépare les produits d'alcoxylation dans la première étape et les esters dans la deuxième étape.

**6.** Produits aqueux pour l'entretien et les soins des cheveux, contenant :
a) de 1 à 10 parties en poids d'au moins l'un des composés de formule générale (1), et
b) de 1 à 20 parties en poids d'au moins un tensio-actif choisi parmi l'ensemble comprenant les tensioactifs non ioniques, amphotères, zwittérioniques et ioniques, et éventuellement
c) de 0,1 à 10 parties en poids d'épaississants, de parfums, de conservateurs, de colorants, d'extraits végétaux et d'autres additifs et adjuvants, et
d) de l'eau jusqu'à 100 parties en poids.

**7.** Shampooing aqueux pour la douche et les cheveux, contenant :
a) de 1 à 10 parties en poids d'au moins l'un des composés de formule générale (1), et
b) de 1 à 20 parties en poids d'au moins un tensio-actif non ionique, amphotère, zwittérionique et ionique, et
c) de 0,1 à 10 parties en poids d'épaississants, de parfums, de conservateurs, de colorants, d'extraits végétaux et d'autres additifs et adjuvants, et éventuellement
d) de l'eau jusqu'à 100 parties en poids.

**8.** Produits aqueux pour le rinçage de la vaisselle, contenant :
a) de 1 à 10 parties en poids d'au moins l'un des composés de formule générale (1), et
b) de 1 à 30 parties en poids d'au moins un tensio-actif choisi parmi l'ensemble comprenant les tensioactifs non ioniques, amphotères, zwittérioniques et ioniques, et éventuellement
c) de 0,1 à 10 parties en poids d'épaississants, de parfums, de conservateurs, de colorants, d'extraits végétaux et d'autres additifs et adjuvants, et éventuellement
d) de l'eau jusqu'à 100 parties en poids.

**9.** Produits pour les soins de la peau, contenant :
a) de 1 à 10 parties en poids d'au moins l'un des composés de formule générale (1), et
b) de 1 à 10 parties en poids d'au moins un tensio-actif choisi parmi l'ensemble comprenant les tensioactifs non ioniques, amphotères, zwittérioniques et ioniques, et éventuellement
c) de 2 à 10 parties en poids d'huiles végétales ou minérales ou d'huiles esters,
d) de 1 à 5 parties en poids d'un agent de consistance
e) de 0,5 à 5,0 parties en poids de parfums, de colorants ou de conservateurs,
f) de l'eau jusqu'à 100 parties en poids.

**10.** Produits pour le nettoyage de la peau, contenant :
a) de 1 à 10 parties en poids d'au moins l'un des composés de formule générale (1), et
b) de 0,1 à 20 parties en poids d'au moins un tensio-actif choisi parmi l'ensemble comprenant les tensio-actifs non ioniques, amphotères, zwittérioniques et ioniques, et éventuellement
c) de 0,1 à 10 parties en poids d'épaississants, de parfums, de conservateurs, de colorants, d'extraits végétaux et d'autres additifs et adjuvants, et éventuellement
d) de l'eau jusqu'à 100 parties en poids.

**Revendications pour l'Etat contractant suivant : ES**

**1.** Procédé pour préparer les composés de formule générale (1)

$$\begin{array}{l} H_2C\text{-}COO\text{-}(CHR\text{-}CH_2\text{-}O)_a\text{-}R^1 \\ \quad | \\ HO\text{-}C\text{-}COO(CHR\text{-}CH_2\text{-}O)_b\text{-}R^2 \\ \quad | \\ H_2C\text{-}COO(CHR\text{-}CH_2\text{-}O)_c\text{-}R^3 \end{array} \qquad (1)$$

dans laquelle :
les radicaux R sont identiques ou différents et représentent chacun H ou $-CH_3$
$R^1$ =

$-CO-CH-CH_2-COO-x^+$ ou $-CO-CH_2-CH-COO-x^+$
|                                      |
$SO_3^-x^+$                            $SO_3^-x^+$

$R^2 = R^1$, ou H, ou $R^3$

$R^3$ est un radical alkyle ou acyle, éventuellement substitué et/ou contenant des doubles liaisons et ayant de 8 à 22 atomes de carbone,

a et b sont identiques ou différents et valent chacun de 0 à 5, avec $a + b \geqq 1$, et

c = 0 à 15, et

$x^+$ est $H^+$ ou est un cation,

caractérisé en ce que dans une première étape, on estérifie le monoester de l'acide citrique avec l'alcool gras ou l'alcool gras alcoxylé, en utilisant éventuellement un catalyseur d'estérification, à une température de 140 à 160 °C tout en éliminant l'eau de réaction ; dans une deuxième étape, on fait réagir ce dernier en présence d'un catalyseur basique à une température de 80 à 150 °C et sous une pression de 1 à 10 bar avec un oxyde d'alkylène pour donner un produit d'alcoxylation, lequel, dans une troisième étape, est mis à réagir, par addition d'anhydride maléique à une température de 60 à 80 °C pour donner le semi-ester, et, dans une dernière étape, à une température de 60 à 80 °C, avec une solution aqueuse de sulfite de sodium pour donner le sel de l'acide sulfonique correspondant.

**2.** Procédé pour préparer des composés de formule générale (1) selon la revendication 1, caractérisé en ce que :

R = H,

$R^1$, $R^2$ =

$-CO-CH-CH_2-COONa-$ ou $-CH-CH_2-CH-COONa-$
|                                      |
$SO_3^-Na^+$                           $SO_3^-Na^+$

$R^3$ est un radical alkyle ayant de 12 à 18 atomes de carbone

a + b = 2 à 5

c = 1 à 10 et

$x^+$ est un cation.

**3.** Procédé pour préparer des composés de formule générale (1) selon la revendication 1, caractérisé en ce que a + b + c = 5 à 15, $R^3$ est un radical acyle ayant de 8 à 18 atomes de carbone, constitué du mélange naturel d'acides gras dérivés de l'huile de coprah.

**4.** Procédé selon les revendications 1 à 3, caractérisé en ce qu'on prépare les produits d'alcoxylation dans la première étape et les esters dans la deuxième étape.

**5.** Produits aqueux pour l'entretien et les soins des cheveux, contenant :

a) de 1 à 10 parties en poids d'au moins l'un des composés de formule générale (1), et

b) de 1 à 20 parties en poids d'au moins un tensioactif choisi parmi l'ensemble comprenant les tensioactifs non ioniques, amphotères, zwittérioniques et ioniques, et éventuellement

c) de 0,1 à 10 parties en poids d'épaississants, de parfums, de conservateurs, de colorants, d'extraits végétaux et d'autres additifs et adjuvants, et

d) de l'eau jusqu'à 100 parties en poids.

**6.** Shampooing aqueux pour la douche et les cheveux, contenant :

a) de 1 à 10 parties en poids d'au moins l'un des composés de formule générale (1), et

b) de 1 à 20 parties en poids d'au moins un tensioactif non ionique, amphotère, zwittérionique et ionique, et

c) de 0,1 à 10 parties en poids d'épaississants, de parfums, de conservateurs, de colorants, d'extraits végétaux et d'autres additifs et adjuvants, et éventuellement

d) de l'eau jusqu'à 100 parties en poids.

**7.** Produits aqueux pour le rinçage de la vaisselle, contenant :

a) de 1 à 10 parties en poids d'au moins l'un des composés de formule générale (1), et
b) de 1 à 30 parties en poids d'au moins un tensioactif choisi parmi l'ensemble comprenant les tensioactifs non ioniques, amphotères, zwittérioniques et ioniques, et éventuellement
c) de 0,1 à 10 parties en poids d'épaississants, de parfums, de conservateurs, de colorants, d'extraits végétaux et d'autres additifs et adjuvants, et éventuellement
d) de l'eau jusqu'à 100 parties en poids.

**8.** Produits pour les soins de la peau, contenant :
a) de 1 à 10 parties en poids d'au moins l'un des composés de formule générale (1), et
b) de 1 à 10 parties en poids d'au moins un tensioactif choisi parmi l'ensemble comprenant les tensioactifs non ioniques, amphotères, zwittérioniques et ioniques, et éventuellement
c) de 2 à 10 parties en poids d'huiles végétales ou minérales ou d'huiles esters,
d) de 1 à 5 parties en poids d'un agent de consistance,
e) de 0,5 à 5,0 parties en poids de parfums, de colorants ou de conservateurs,
f) de l'eau jusqu'à 100 parties en poids.

**9.** Produits pour le nettoyage de la peau, contenant :
a) de 1 à 10 parties en poids d'au moins l'un des composés de formule générale (1), et
b) de 0,1 à 20 parties en poids d'au moins un tensioactif choisi parmi l'ensemble comprenant les tensioactifs non ioniques, amphotères, zwittérioniques et ioniques, et éventuellement
c) de 0,1 à 10 parties en poids d'épaississants, de parfums, de conservateurs, de colorants, d'extraits végétaux et d'autres additifs et adjuvants, et éventuellement
d) de l'eau jusqu'à 100 parties en poids.